(19) Europäisches Patentamt
European Patent Office
Office européen des brevets

(11) Publication number: **0 062 388**
**B1**

# (12) EUROPEAN PATENT SPECIFICATION

(45) Date of publication of patent specification: **04.09.85**

(51) Int. Cl.[4]: **C 02 F 3/28,** A 01 C 3/02

(21) Application number: **82200413.1**

(22) Date of filing: **01.04.82**

## (54) A method and installation for anaerobic fermentation of liquid manure.

(30) Priority: **03.04.81 NL 8101682**

(43) Date of publication of application:
**13.10.82 Bulletin 82/41**

(45) Publication of the grant of the patent:
**04.09.85 Bulletin 85/36**

(84) Designated Contracting States:
**AT BE CH DE FR GB IT LI LU NL SE**

(56) References cited:
**DE-A-2 607 114**
**DE-B-1 122 002**
**DE-C- 907 280**
**GB-A-1 513 416**
**GB-A-1 567 578**
**NL-A-8 001 072**
**US-A-2 720 489**

**JOURNAL OF WATER POLLUTION CONTROL FEDERATION, vol.45, no.11, November 1973, Washington D.C., (US), A.T.EL-SHAFIE et al.: "Anaerobic treatment in a multiple upflow filter system", pp. 2345-2357**

(73) Proprietor: **De Erven G.de Boer B.V.**
**Jupiterweg 17**
**NL-8938 AD Leeuwarden (NL)**

(72) Inventor: **van Staveren, Nicolaas Arie**
**Daverzicht 11 Daver**
**NL-4012 BL Kerk-Avezaath (NL)**

(74) Representative: **Mathol, Heimen**
**EXTERPATENT Willem Witsenplein 4**
**NL-2596 BK 's-Gravenhage (NL)**

**EP 0 062 388 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a bio-gas producing installation for anaerobic fermentation of a liquid suspension of an organic substance and the production of methane gas therefrom, comprising a treatment chamber with an inlet means for influent and an outlet means for the gas and the effluent.

Such an installation is known from GB—A—1 567 578. The mixture is recirculated which results in an intersification of the fermentation. The residence time of the suspension within the treatment chambers will be determined by an attunement between the capacity of both treatment chambers on the one hand and the quantity of influent supplied per unit of time on the other hand. After a certain run-in period, there arises an equilibrium during which fermentation has entirely or largely occurred, whereupon the effluent can be subjected to an after treatment in a conventional manner. The efficiency of the process is, to a large extent, due to the size of the surface of the contact media upon which settles a film of an anaerobic-bacteria culture. This British Specification is however silent about the structural features required for an efficient operation. The invention aims at filling that gap.

According to the invention, the bio-gas producing installation is characterized in that the treatment chamber is composed of two concentric vessels, the central vessel being formed by an upwardly diverging cone filled with contact media, the open upper end of said cone freely communicating with the outer vessel which is at the bottom provided with the inlet means for the influent and at the top with a gas-discharge means, the outer vessel being connected with a standpipe for discharging the effluent, while a pump connection is provided between the bottom of the outer vessel and the point of the central vessel. In the central vessel, a considerable flow rate is imparted to the liquid mixture, due to the presence of the contact media. As a result thereof, there arises a labyrinth structure having a strong turbulence in the liquid, thus bringing the bacterial culture on the contact media and the organic substance of the mixture into intimate contact with one another. This intensive contact and the optimum mixing of the active sludge with the influent are the cause of the very short decomposition time of the organic material that can be obtained in this installation.

It is a further object of the invention to raise the efficiency of the installation by proposing a particular manner in which it is used in a larger entity. According to the invention, the mixture passes through several standard installations of the type herein before described, which are connected in series and each composed of two concentric treatment vessels in which the mixture is recirculated. This results in two important advantages, i.e. firstly, it reduces to a considerable extent a premature discharge of mixture parts that have not been completely fermented as yet, since each part of the mixture must pass through all the standard installations and thus will have sufficient opportunity to attain the optimum degree of fermentation. Secondly, by using a plurality of smaller installations (reactors) the method can be adapted inexpensively to the quantity presented of the mixture to be treated. Consequently, there is no need to manufacture the treatment chambers in all sorts of sizes.

The invention will be further explained with reference to the drawing illustrating a complete bio-gas-producing installation according to the invention and parts thereof.

Fig. 1 is a very schematic view of the most important parts of the entire installation;

Fig. 2 is a vertical, longitudinal sectional view of the bio-gas reactor of the installation of Fig. 1;

Figs. 3—5 are sectional views taken on lines II—II, III—III and IV—IV in Fig. 2.

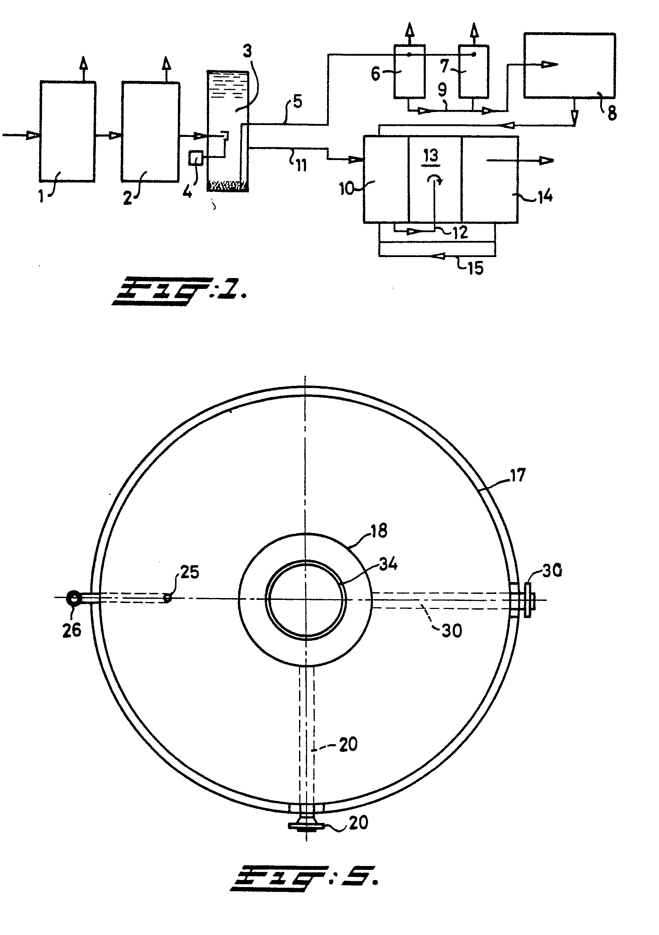

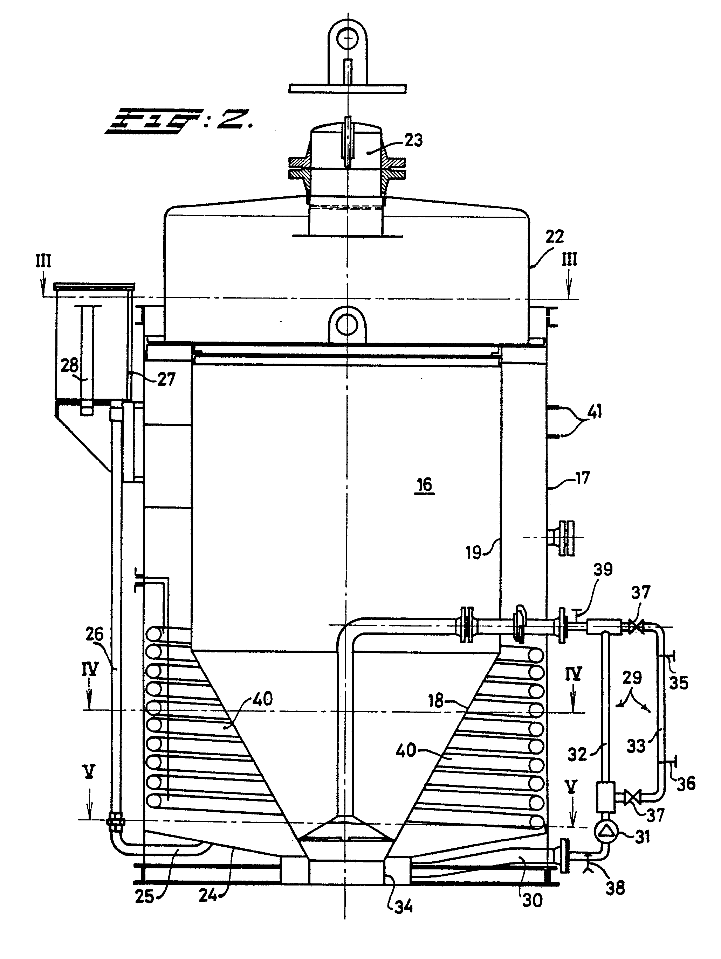

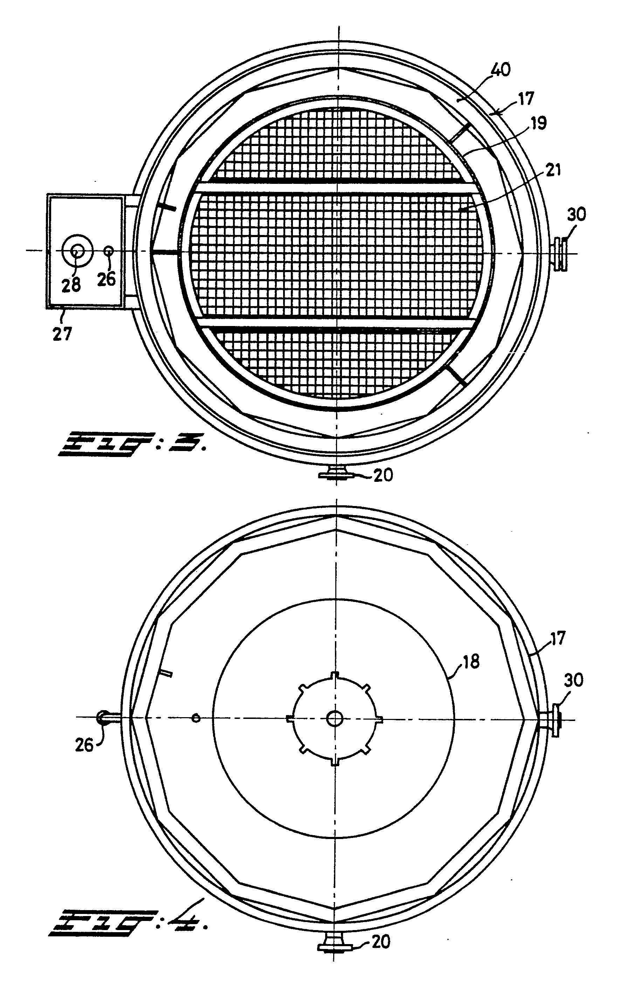

The complete installation as shown in Fig. 1 may consist of a number of components known per se, combined with two bio-gas reactors 1 and 2 which each form a standard unit to be discussed with reference to Figs. 2—5. The complete installation may further comprise a flocculator 3 having a metering pump 4, in which case there occurs in the vessel 3 a flocculation as well as a settling. The sludge settled passes, via a conduit 5, to two sludge-fermentation columns 6 and 7 provided with a sieve bottom.

Through a conduit 9, the effluent is carried from the columns 6 and 7 via a drying tank 8 back to a prestorage chamber 10, to which chamber effluent is also supplied from the flocculator 3 via a conduit 11. The effluent subsequently passes, via a conduit 12, from the chamber 10 to an anaerobic reactor 13 and is subsequently subjected to denitrification in a chamber 14. For the purpose of removing sediment, if any, in the chamber 14, there is provided a return line 15 leading to the prestorage chamber 10. Thus, the effluent as finally obtained can have reached a degree of purity such, as to afford its disposal into an existing sewer system or duct.

The method according to the invention proceeds within the reactor units 1 and 2. Such a unit is shown more in detail in Fig. 2 and consists of a treatment chamber composed of two concentric vessels 16 and 17. The central vessel 16 is formed by an upwardly diverging cone 18 and a cylindrical part 19. The central vessel 16 is filled with contact media (not shown), for example plastics rings. The central vessel 16 freely communicates at its open top with the outer vessel 17, whose bottom is provided with an inlet means 20, to be seen in Fig. 5. The top end of vessel 16 is provided with a grate 21, vide Fig. 3, for preventing displacement of the contact media. On the vessel 17 there is fitted a bell-shaped gas hood or gas bag 22 having an outlet means 23.

The outer vessel 17 has a somewhat tapered bottom 24, a pipe 25 being connected thereto which merges into a standpipe 26 leading to a reservoir 27. A second standpipe 28 ensures the maintaining of a certain level of liquid within the reservoir 27, thus providing counterpressure for the overpressure of the bio-gas being formed in the hood of bag 22.

Between the bottom 24 of the outer vessel 17 and the downwardly directed point of the cone 18 there is provided a pump connection 29. This connection consists of a suction line 30, a pump 31, a main pipe 32, and a shunt 33. Both the inlet means 20 and the line 30 are connected to a central recessed portion 34 of the bottom 24 of the outer vessel 17.

In the pump connection 29, the measuring means 35 and 36 (electrodes) are located in the shunt 33 for measuring the composition of the liquid mixture circulating through the reactor vessels 16 and 17. The shunt 33 is provided at both ends with a shut-off valve 37. In the suction line 30 there are provided metering points 38 (very schematically indicated) for basic substances (soda lye or soda) and for glucose ($C_6H_{12}O_6$). In the uppermost part of the main pipe 32 there is provided a thermostat 39 for a purpose still to be described hereinafter.

Inside the vessel 17 there is provided a heat exchanger 40 for heating or cooling the mixture present in the reactor as a function of the prevailing ambient temperature. To ensure an optimum operation, it is necessary to maintain a constant temperature inside the reactor at a given level, and this may sometimes require a heating or cooling of the mixture. Controlling the heat exchanger 40 proceeds by means of the thermostat 39 provided in the pump connection 29. In the casing of the vessel 17, in the vicinity of the top, a few measuring devices 41 are also provided for measuring the pH value. Said means are connected to the metering points 38 provided in the suction line 30.

As is apparent from the foregoing, the treatment installation as shown in Fig. 2 consists of an upflow reactor 16 with fitted therein a filling of plastics rings having a very large contact surface. This reactor 16 is disposed in a downstream precipitation vessel 17, a gas hood or gas bag 22 being mounted on top of the whole, conceivably in conjunction with a foam separator. The pump 31 sucks the liquid to be treated from the deepened portion 34, where also the influent is admitted via the inlet means 20. Subsequently, the pump 31 forcefully pumps the liquid up from below through the upflow reactor 16 and past the plastics rings to the down-stream precipitation vessel 17. By continuously supplying the influent, the liquid level rises upwards, whereupon said liquid can be discharged to a subsequent stage via the overflow 25—28. The gas production can be controlled by varying the flow of influent through the inlet means 20 as well as by the temperature. The temperature regulation may be coupled to a weather-dependent control causing the reactor temperature to rise in the event of a decreasing ambient temperature, and causing it to be decreased in the event of an increasing ambient temperature. It is then important to also simultaneously adjust the flow of influent. The heat exchanger 40 for bringing the installation at the proper temperature may be disposed within the vessel 17, but may also be provided on the outside thereof, if necessary, for exmaple in the pump connection 29.

After its residence time in the reactor units, the effluent can still be fermented subsequently in a second-stage reactor or be mixed with a flocculant in a clarifier 3. The mixture so obtained may be supplied to a flocculation tank (not shown), whereupon the sludge can be separated from the effluent or can be supplied to a sludge-fermentation column 6, 7 having a sieve bottom, in which case the sludge separation can be carried out during a considerable residence time.

During operation, the pH is measured with the means 41 and, if necessary, corrected by means of a dosage of soda lye or soda, while adding at the same time a small quantity of glucose. This dosage of glucose has proved to be of essential importance, since the composition of floating manure has a shortage of certain ions, which is thus eliminated.

In the upflow reactor 16, there is produced a high liquid-flow rate due to the presence of the plastics rings which bring about, as it were, a labyrinth structure. The liquid flow acquires a strongly turbulent character, bringing the bacterial culture on the plastics rings and the organic substance of the mixture into intimate contact with one another. This very intensive contact and the optimum mixing of active sludge with the influent affords achieving a very short decomposition time (12—24 hours) of the organic material to be obtained. It is also important to note that for the purpose of purifying the effluent by means of flocculation, proper decomposition of the influent is absolutely required prior to reaching the subsequent stage.

During the gas production under the influence of the increased temperature, water vapour will be produced as well. Using a non-insulated gas hood or gas bag 22 will enable this vapour to be liquified and to flow back into the mixture. By using the installation according to the invention it has proved possible to limit the fermentation time of floating pig manure to 24 hours, whereafter flocculation can be carried out. The capacity of the installation of Fig. 2 is, for instance, 8 $m^3$, in which case the mixture has then passed about 150 times through the upflow reactor 16.

The total contact surface in the vessel 16 is approximately 100 $m^2$, which entails that every 24 hours the liquid comes into contact with 16,000 $m^2$ of surface. This equals a liquid film having a thickness of 0,4 mm per 24 hours.

The following data can still be given with respect to the embodiment provided above:

| | |
|---|---|
| COU (chemical oxygen use) | 100,000 mg/l |
| BOU (biological oxygen use) | 33,000 mg/l |

| | |
|---|---|
| NKj (Nitrogen according to KJELDAL) | 7,200 |
| $NH_4OH$ (ammonia) | 4,500 |
| P (phosphate) | 2,200 |
| residue after evaporation | 80,000 mg/l |
| ashes | 22,000 mg/l |
| gas production $CH_4$ | 35 $m^3$ |
| N | 7 $m^3$ |
| fermentation temperature | 35—37°C |
| residence time | 24 hours |
| addition of glucose | 5 kg/$m^3$ influent. |

The method and the installation according to the invention are useful not only with a bio-gas-producing system, but also for the aftertreatment of the effluent of a waste-water purification process. The installation can also be used for the pretreatment of surface water on behalf of the drinking-water supply. Among the further possibilities may be cited the denitrification of waste water, the preparation of methanol from straw pulp or wood pulp and from waste paper, and finally a.o. water treatment in fish nurseries. The installation according to the invention can furthermore be used for anaerobic purification of waste water from slaughter houses, starch production facilities, the preserves industry, the paper industry, the dairy industry, etc. It is obvious that the scope of the present invention is not limited to the above possibilities.

It is observed that the reference numerals in the claims are not intended to restrict the scope thereof, but are only denoted for clarification.

**Claims**

1. A bic-gas-producing installation for anaerobic fermentation of a liquid suspension of an organic substance and the production of methane gas therefrom, comprising a treatment chamber with an inlet means for influent and an outlet means for the gas and the effluent, characterized in that the treatment chamber is composed of two concentric vessels (16, 17), the central vessel being formed by an upwardly diverging cone (18) which is filled with contact media, the open upper end of said cone freely communicating with the outer vessel (17), whose bottom (24) is provided with an inlet means (20) for the influent and whose top is provided with a gas-discharge means (23), the outer vessel (17) being connected with a standpipe (26, 28) for discharging the effluent, a pump connection (29) being provided between the bottom (24) of the outer vessel (17) and the point of the cone (18).

2. An installation according to claim 1, characterized in that the pump connection (29) is formed by a suction line (30) which leads to the point of admission (20) for the influent at the bottom (24) of the outer vessel (17) and which is connected to a pump (31) and a pressure line (33) through which the mixture of the organic substance and the active sludge is discharged into the interior of the downwardly directed point (18) of the central vessel (16), means (35, 36) being provided in said line for measuring the composition and pH of the mixture and for metering substances to be added.

3. An installation according to claim 1 or 2, characterized in that the central vessel (16) is at its top provided with a cylindrical skirt (21) forming an overflow edge, and in that the bottom of the outer vessel has a central recessed portion (34) into which extend both the influent inlet means (20) and the suction line (30).

4. An installation according to any one of claims 1—3, characterized in that the contact media located within the central vessel (16) are provided with a coating of active carbon.

5. An installation according to any one of claims 1—4, characterized in that a heat exchanger (40) is provided, the pump connection (29) comprising a thermostat (39) for measuring the temperature of the circulating mixture, for controlling therewith the heat exchanger so as to compensate for the heat transfer in the concentric vessels (16, 17) resulting from the ambient temperature.

6. A method for anaerobic fermentation of a liquid suspension of an organic substance and the production of methane gas therefrom, the suspension (the influent) being mixed with active sludge and kept in movement, while furthermore the produced gas is separated and the liquid thus produced (the effluent) is discharged for possible subsequent treatment, in which the suspension is supplied to a collecting point for the active sludge, and this mixture of organic substance and sludge is continuously recirculated through at least two treatment chambers disposed in series, an upwardly directed flow occurring through the first chamber in the presence of a close assembly of contact media thus causing a strong turbulence to be produced in the mixture, while furthermore a downwardly directed flow flows through the second chamber, which serves as a precipitation vessel provided with an outlet means for the effluent, characterized in that the mixture passes through several standard installations of the type according to claim 1 which are connected in series and each composed of two concentric treatment vessels in which the mixture is recirculated.

7. A method according to claim 6, characterized in that the recirculation of the mixture is continued until the effluent can be subsequently treated in a flocculator followed by a clarifier.

**Patentansprüche**

1. Anlage zur Gewinnung von Biogas durch anaerobe Gärung einer flüssigen Suspension eines organischen Stoffes und zur Herstellung von Methangas daraus, mit einem Behandlungsraum, der eine Einlaßeinrichtung für den Zufluß und eine Auslaßeinrichtung für das Gas und den Abfluß aufweist, dadurch gekennzeichnet, daß der Behandlungsraum aus zwei konzentrischen Behältern (16, 17) besteht, daß der Innenbehälter (16) durch einen nach oben sich öffnenden, mit Kontaktkörpern gefüllten Kegel (18) gebildet wird, dessen offenes oberes Ende in freier Verbindung mit dem Außenbehälter (17) steht, dessen Boden (24) mit einer Einlaßeinrichtung (20) für den Zufluß und dessen Oberteil mit einer Gasauslaßeinrichtung (23) versehen ist, daß sich an dem Außenbehälter (17) ein Steigrohr (26, 28) für die Abgase des Abflusses befindet, und daß zwischen dem Boden (24) des Außenbehälters (17) und der Spitze des Kegels (18) eine Pumpenverbindung (29) vorgesehen ist.

2. Anlage nach Anspruch 1, dadurch gekennzeichnet, daß die Pumpenverbindung (29) durch eine Saugleitung (30) gebildet ist, die zu der Zuflußzuführung (20) am Boden (24) des äußeren Gefäßes (17) führt und die mit einer Pumpe (31) und einer Druckleitung (33) in Verbindung steht, durch welche das Gemisch aus dem organischen Stoff und dem aktiven Schlamm in das Innere der abwärts gerichteten Spitze des Kegels (18) des Innenbehälters (16) abgegeben wird, und daß in diesr Leitung eine Einrichtung (35, 36) zum Bestimmen der Zusammensetzung und des pH des Gemischs und zum Dosieren von zuzufügenden Stoffen vorgesehen ist.

3. Anlage nach Anspruch 1 oder 2, dadurch gekennzeichnet, daß der Innenbehälter (16) an seinem oberen Ende mit einem zylindrischen Rand (21) versehen ist, der eine Überlaufkante bildet, und daß der Boden des Außenbehälters eine mittige Aussparung (34) aufweist, in die die Zuflußzuführung (20) und die Saugleitung (30) hineinragen.

4. Anlage nach einem der Ansprüche 1 bis 3, dadurch gekennzeichnet, daß die Kontaktkörper in dem Innenbehälter (16) mit einem Überzug von Aktivkohle versehen sind.

5. Anlage nach einem der Ansprüche 1 bis 4, dadurch gekennzeichnet, daß ein Wärmeaustauscher (40) vorgesehen ist, und daß die Pumpenverbindung (29) einen Thermostaten (39) zum Messen der Temperatur des umlaufenden Gemischs aufweist, um dadurch den Wärmeaustauscher so zu steuern, daß die durch die Umgebungstemperatur hervorgerufene Wärmeübertragung in den konzentrischen Behälter (16, 17) ausgeglichen wird.

6. Verfahren zur anaeroben Gärung einer flüssigen Suspension eines organischen Stoffes und zur Gewinnung von Methangas daraus, wobei die Suspension (der Zufluß) mit aktivem Schlamm vermischt und in Bewegung gehalten wird, während außerdem das gewonnene Gas abgetrennt und die auf diese Weise gewonnene Flüssigkeit (der Abfluß) für eine etwaige Weiterbehandlung abgegeben wird, wobei ferner die Suspension einem Sammelpunkt für den aktiven Schlamm zugeführt wird und dieses Gemisch aus organischem Stoff und Schlamm ständig durch mindestens zwei hintereinanderliegende Behandlungsräume umgepumpt wird, und wobei schließlich ein aufwärts gerichteter Strom durch den ersten Raum in Gegenwart einer dichten Ansammlung von Kontaktkörpern fließt, so daß in dem Gemisch eine starke Turbulenz erzeugt wird, während außerdem ein abwärts gerichteter strom durch den zweiten Raum fließt, der als Absitzbehälter dient und eine Auslaßeinrichtung für den Abfluß aufweist, dadurch gekennzeichnet, daß das Gemisch durch einige Standardanlagen der in Anspruch 1 beschriebenen Art fließt, die hintereinandergeschaltet sind und jeweils aus zwei konzentrischen Behandlungsbehältern bestehen, in denen das Gemisch umgepumpt wird.

7. Verfahren nach Anspruch 6, dadurch gekennzeichnet, daß das Umpumpen des Gemischs fortgesetzt wird, bis der Abfluß anschließend in einem Flockungsbehälter und nachgeschalteten Klärbehälter behandelt werden kann.

**Revendications**

1. Installation produisant du bio-gaz pour la fermentation a noérobie d'une suspension liquide d'une substance organique et la production du méthane gazeux à partir de celle-ci, comprenant une chambre de traitement avec un moyen d'entrée pour l'influent et un moyen de sortie pour le gaz et l'effluent, caractérisée en ce que la chambre de traitement est composés de deux récipients concentriques (16, 17), le récipient central étant formé par un cône (18) s'élargissant vers le haut et qui est rempli des milieux de contact, l'extrémité supérieure ouverte de ce cône communicant librement avec le récipient extérieur (17), dont le fond (24) est muni d'un moyen d'entrée (20) pour l'influent et dont le haut est muni d'un moyen de dégagement de gaz (23), le récipient extérieur (17) étant relié au tuyau de montée (26, 28) pour la décharge de l'effluent, un raccord de pompe (29) étant disposé entre le fond (24) du récipient extérieur (17) et l'extrémité du cône (18).

2. Installation suivant la revendication 1, caractérisée en ce que le raccord de pompe (29) est formé par un conduit d'aspiration (30) qui mène au point d'admission (20) de l'influent au fond (24) du récipient extérieur (17) et qui est relié à une pompe (31) et à une conduite sous pression (33), à travers laquelle est déchargé le mélange de substance organique et de boue activée dans l'intérieur de la pointe (18) dirigée vers le bas du récipient central (16), des moyens étant disposés dans ce conduit pour déterminer la composition et le pH du mélange et pour mesurer les substances à ajouter.

3. Installation suivant la revendication 1 ou 2, caractérisée en ce que le récipient central (16) comprend à sa partie supérieure une chemise cylindrique (21) formant un bord déversoir et que le fond du récipient extérieur présente une position centrale en retrait (34), dans laquelle se prolongent à la fois le moyen (20) d'entrée de l'influent et la conduite d'aspiration (30).

4. Installation suivant l'une quelconque des revendications 1 à 3, caractérisée en ce que les milieux de contact situés à l'intérieur du récipient central (16) sont pourvus d'un revêtement de charbon actif.

5. Installation suivant l'une quelconque des revendications 1 à 4, caractérisée en ce qu'elle comprend un échangeur de chaleur (40), un reccord de pompe (29) comprenant un thermostat (39) pour mesurer la température du mélange circulant et contrôler ainsi l'échangeur de chaleur de façon à compenser le transfert thermique dans les récipients concentriques (16, 17) résultant de la température ambiente.

6. Procédé de fermentation anoérobie d'une suspension liquide d'une substance organique et la production d'un méthane gazeux à partir de celle-ci, la suspension (l'influent) étant mélangée à la boue activée et maintenue en mouvement, tandis que le gaz produit est séparé et le liquide ainsi produit (l'effluent) est décharge pour un éventuel traitement ultérieur, dans lequel la suspension est fournie à un point collecteur pour la boue activée et ce mélange de substance organique et de boue est recyclé en continu à travers deux chambres de traitement au moins disposées en série, un courant montant apparaissant dans la première chambre en présence d'un assemblage étroit de milieux de contact provoquant ainsi une forte turbulence dans le mélange, tandis que de plus en plus, un courant descendant s'écoule à travers la deuxième chambre qui sert de récipient de précipitation pourvu d'un moyen de sortie pour l'effluent, caractérisé en ce que le mélange traverse plusieurs installations standards du type qui est décrit dans la revendication 1, qui sont reliées en série et composée chacune de deux récipients du traitement concentriques dans lesquels est recyclé le mélange.

7. Procédé suivant la revendication 6, caractérisé en ce que le recyclage du mélange est poursuivi jusqu'à ce que l'effluent puisse être traité ultérieurement dans un floculateur, puis un clarificateur.

1
2
3
4
5
6
7
8
9
10
11
12
13
14
15
Fig: 1.

17
18
34
25
26
30
30
20
20
Fig: 5.

Fig: 2.
23
22
III
III
28
27
41
16
17
19
39
37
26
IV
IV
40
18
35
29
33
40
32
36
V
V
37
31
25
24
34
30
38

40
17
19
21
30
28
26
27
Fig: 3.
20
17
18
30
26
Fig: 4.
20